# EUROPEAN PATENT APPLICATION

(11) **EP 1 870 104 A1**
(43) Date of publication of application: **26.12.2007**
(21) Application number: 06115571.9
(22) Date of filing: 16.06.2006
(51) Int. Cl.: A61K 31/715, A61K 31/7008, A61K 45/00, A61K 31/20, A61K 36/9068, A61K 36/82, A61K 35/20, A61P 19/02

(54) **Composition for the relief of joint pain**

(71) Applicant: Nestec S.A., 1800 Vevey (CH)
(72) Inventor: Annett, Philip, 1143 Apples (CH)
(74) Representative: Dixon, Sarah

(57) **Abstract**

The invention relates to a composition suitable for the relief of joint pain in athletes comprising a food grade anti-inflammatory substance and chondroitin sulphate or glucosamine. The invention further extends to a method for the prevention or treatment of joint pain in athletes which comprises administering to the athlete a food grade anti-inflammatory substance and chondroitin sulphate or glucosamine.

## Description

### Field of the Invention

This invention relates to the prevention and treatment of joint pain.

### Background of the Invention

Joint pain can be caused by many types of injuries or conditions. The most common joint disorder is osteoarthritis which affects 70% of the population over 65 years. However, another group that often suffers from joint pain is athletes practising impact sports such as running, football etc. In this group, incidence and severity of joint pain does not appear to be directly linked to age.

The cause or causes of joint pain in athletes are currently not well understood. It is possible that frequent prolonged weight-bearing exercise may damage the cartilage which is a thin layer of white soft tissue which covers the ends of bones inside the articulating joints of the body. Together with the synovial fluid, the covering of cartilage reduces the level of friction between opposing bones in a joint almost to zero as well as dissipating mechanical energy so that the joint structure is protected from damage by impact forces. It is known that progressive degeneration of articular cartilage is a feature of the development of age-related osteoarthritis.

However, inflammation of the joint and the resultant pain and swelling generally only occur in osteoarthritis in the later stages of the disease. Indeed, many individuals who suffer from osteoarthritis are not aware of the problem until the disease reaches a late stage because it is not until this stage that inflammation occurs and produces noticeable symptoms. By contrast, pain in joints such as the knees and ankles is often the first symptom noticed by athletes suffering from joint problems.

Treatment for joint pain in athletes has generally focused on relieving the pain and other symptoms of inflammation and non-steroidal anti-inflammatory drugs such as aspirin and ibuprofen are widely prescribed for this purpose. However, consumption of these medicaments over a prolonged period as may be necessary in the treatment of joint pain can have side effects such as stomach upsets, abdominal pain and even ulceration in severe cases. Further, the treatment is restricted to alleviation of the symptoms and does nothing to improve the underlying condition.

It has been shown that glucosamine sulphate orally administered is absorbed and distributed to joint tissue and may reduce swelling and improve mobility in patients with osteoarthritis. However, the time taken before the patient sees an improvement is relatively long as alleviation of the symptoms relies on increased or improved cartilage synthesis.

Therefore, there remains a need for a method to prevent and treat joint pain in athletes which will provide both quick and permanent relief from the pain without undesirable side effects.

### Summary of the Invention

The present inventors have surprisingly found that the co-administration of a food grade anti-inflammatory substance and a cartilage precursor is particularly effective in the prevention and treatment of joint pain in athletes.

Accordingly, in a first aspect, the present invention provides a composition suitable for the relief of joint pain in athletes comprising a food grade anti-inflammatory substance and chondroitin sulphate or glucosamine.

In a second aspect, the present invention provides the use of a food grade anti-inflammatory substance and chondroitin sulphate or glucosamine in the manufacture of a medicament or therapeutic nutritional composition for the prevention or treatment of joint pain in athletes.

The invention extends to a method for the prevention or treatment of joint pain in athletes which comprises administering to an athlete in need thereof a therapeutic amount of a food grade anti-inflammatory substance and chondroitin sulphate or glucosamine.

### Detailed Description of the Invention

In the present specification, the following words are given a definition that must be taken into account when reading and interpreting the description, examples and claims.

"food grade anti-inflammatory substance" means a substance with anti-inflammatory substance which either has a long history of safe use in food or beverage products for consumption by humans or which has been accorded GRAS status or similar by the FDA or other official regulatory body.

All percentages are by weight unless otherwise stated.

The food grade anti-inflammatory substance and glucosamine or chondroitin sulphate may be administered using the same carrier or may be administered sequentially.

Examples of suitable food grade anti-inflammatory substances include omega-3 fatty acids such as docosahexaenoic acid and eicosapentaenoic acid, ginger extract, green tea polyphenols and anti-inflammatory milk fractions. A preferred anti-inflammatory milk fraction is the whey fraction sold by Stolle Milk Biologics Inc under the trade mark MicroLactin®. This fraction is produced by the process described in EP 336694, the contents of which are incorporated herein by reference. Preferably the daily dose of MicroLactin is between 1 and 6 grams per day, more preferably between 1 and 4 grams per day. The amount to be included in a composition according to the invention will be selected depending upon the nature of the composition and how it is to be administered. For example, if the composition is to be administered twice a day, each supplement may contain from 0.5 to 3 grams MicroLactin.

Glucosamine is an amino sugar synthesised from glucose which is used in the synthesis of glycosaminoglycans (GAGs). GAGs are a major constituent of articular cartilage where they are found as a constituent of proteoglycans which constitute part of the amorphous gel which is an important component of articular cartilage. It may be administered as glucosamine sulphate, an artificially synthesised sulphate salt of 2-amino-2-deoxy-D-glucopyranose. Preferably, the daily dose of glucosamine is between 1 and 5 grams per day. Again, the amount to be included in a composition according to the invention will be selected depending upon the nature of the composition and how it is to be administered.

Chondroitin sulphate is a mono-sulphated GAG consisting of repeating disaccharide (N-acetylgalactosamine and glucuronic acid) units. It may be administered as a mixture of chondroitin 4- and 6- sulphates derived from bovine or shark cartilage. Preferably, the daily dose of chondroitin sulphate is between 1 and 8 grams per day. Again, the amount to be included in a composition according to the invention will be selected depending upon the nature of the composition and how it is to be administered.

Compositions according to the invention may contain glucosamine or chondroitin sulphate or both.

The composition according to the invention may be a food composition for human consumption. This composition may be a nutritionally complete formula, a dairy product, a chilled or shelf stable beverage, a soup, a dietary supplement, a meal replacement, or a nutritional bar for example. Product formats particularly suitable for use by athletes include nutritional bars based on cereals, nutritional bars based on proteins and nutritional supplements in gelled form.

A particularly preferred food composition for use in the present invention is a nutritional bar based on a mixture of cereals. This is a formulation which is particularly attractive to athletes as it is durable, easily portable and can be made in different flavours to avoid the onset of taste fatigue. Furthermore, it is believed that such formulations provide a particularly suitable carrier for the active ingredients in terms of the efficiency of their absorption by the body after ingestion.

Cereals include wheat, maize, barley, oat, rice, oat, millet and the like. Cereals may be included as flours but also in the form of crisps, flakes, puffs (oven or gun puffed), extruded and/or extruded-expanded grains. The cereal or cereals to be used will be chosen depending on the desired density of the final bar. For example, if a light bar is preferred, it is better to include cereals as crisps and/or puffs, whereas if a dense bar is preferred, it may be better to use flakes or baked and compressed cereals, or simply flour, such as rice flour, for example.

If high amounts of oat bran and/or oat bran concentrate are used (in order to provide β-glucan for example), other cereals may be omitted completely.

Apart from the use of cereals as a base, the other components of the bar are not thought to be of particular importance to the invention. Many compositions for, and methods of manufacture of, such bars are known.

The bar may also comprise nuts. Examples are hazelnuts, walnuts, pecan nuts, cashew nuts, almonds, coconut, chestnut, macadamia nuts and mixtures of these. Nuts may be present in amounts up to 15%, preferably up to 10% by weight of the dry ingredients. Dried fruits, such as apple, peach, pear, apricot, banana, orange and pineapple and/or berries, such as raspberry, strawberry, blackberry, blueberry and the like may also be incorporated.

The bar may be prepared by mixing the dry ingredients, which may include cereals, soluble fibre, nuts, fruits, chocolate, berries, and milk solids with a binder, which is usually in the form of a syrup. The dry ingredients will usually comprise 40 to 90%, preferably 60 to 80% by weight of the total recipe, with the binder accounting for the balance (60 to 30%, preferably 50 to 40% by weight).

The binder may be a glucose syrup comprising a mixture of glucose and its polymers obtained by partial hydrolysis of starch and having a DE (dextrose equivalent) of about 30 to 50 and a water content of from about 15 to 25% by weight of the binder. The binder may also include invert sugar and/or high fructose corn syrup, for example.

At its simplest, the binder may be a syrup comprising water and sugars. It may also include fat (up to 15%, preferably 2 to 10%), lecithin (up to 1%, preferably 0.01 to 0.2%) and/or flavours. The binder may be prepared by heating the water to 70 to 90°C and adding the sugars with stirring. The resulting syrup may then be cooled and other constituents added as desired. Syrups with a water content of about 15 to 20% by weight are also commercially available.

The binder may comprise up to 15%, preferably 0.5 to 5% glycerol. This enables a bar with a moist mouthfeel to be provided at a low water content, thus enhancing shelf-life.

The binder may comprise milk solids, in the form of milk powder and/or fresh milk. In the latter case, the addition of water may be at least partially replaced by the addition of milk. Such a binder is described in WO 00/56171 and comprises 10 to 70 parts of sugar, 0.5 to 5 parts of a binding agent (a polysaccharide such as a gum), up to 15 parts of glycerol, up to 60 parts of fruit pulp or concentrate, up to 10 parts of cocoa powder and added water up to a water content of from 10 to 30%.

The food grade anti-inflammatory substance and glucosamine or chondroitin sulphate may be added to the binder or the dry ingredients as preferred.

The dry ingredients and the binder may be mixed in any suitable mixing apparatus know in the art such as a screw mixer of the helical spring type with an axial sprinkling nozzle or with a coating drum, for example.

The mass thus formed may be transferred into a suitable forming or pressing apparatus, to shape the bar. For example, a Bepex-Hutt Roller Press type DP in which the mass is pushed through a slab or strand nozzle under pressures of up to 12 bar (preferably 5 to 10 bar) may be used. Another suitable apparatus is the Bepex-Hutt Roller Slab Former type GP, which uses lower high pressures and therefore produces a flat paste with a lower density.

Alternatively, the bars may be obtained by rolling or pressure rolling, transfer on a pressure band, pre-cooling (10 to 20°C) and cutting of the final shape, for example, by a slitter or guillotine cutter followed by a second cooling (4 to 15°C), and final packaging of the bar.

In another embodiment, the composition may be a supplement including the food grade anti-inflammatory substance and the glucosamine or chondroitin sulphate in an amount sufficient to prevent or treat joint pain in an individual. As with a nutritional composition, the amounts of these substances to be included in the supplement will be selected accordingly depending upon how may times per day the supplement is to be administered. The supplement may be in the form of tablets, capsules, pastilles or a liquid for example. The supplement may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellifying agents and gel forming agents. The supplement may also contain conventional pharmaceutical additives and adjuvants, excipients and diluents, including, but not limited to, water, gelatine of any origin, vegetable gums, ligninsulfonate, talc, sugars, starch, gum arabic, vegetable oils, polyalkylene glycols, flavouring agents, preservatives, stabilizers, emulsifying agents, buffers, lubricants, colorants, wetting agents, fillers, and the like.

Further, the supplement may contain an organic or inorganic carrier material suitable for oral or enteral administration as well as vitamins, minerals trace elements and other micronutrients in accordance with the recommendations of Government bodies such as the USRDA.

### Example

An example of the composition of a cereal bar according to the present invention is given below. This composition is given by way of illustration only.

| **Ingredient** | **Batch Load Proportion** | | **Solids Proportion** |
|---|---|---|---|
| High fructose corn syrup | 41.910 | | 36.043 |
| Oat bran | 16.6300 | | 16.430 |
| Maltodextrin | 9.720 | | 9.331 |
| Milk protein isolate | 7.700 | | 7.392 |
| Rice flour | 6.070 | | 5.706 |
| Cereal crisp | 6.070 | | 5.936 |
| Almond paste | 3.480 | | 3.410 |
| Vitamin and mineral premix | 2.800 | | 2.674 |
| MicroLactin® | 2.300 | | 2.162 |
| Glucosamine sulphate | 2.300 | | 2.300 |
| Vanilla flavour | 1.020 | | 0.959 |

| | | | |
|---|---|---|---|
| **Composition** | | | |
| | g/100g | Kcal/100g | Kcal % |
| Water | 7.656 | | |
| Available carbohydrates | 66.529 | 266.116 | 75.807 |
| Protein | 13.042 | 52.168 | 14.861 |
| Fat | 3.640 | 32.760 | 9.332 |
| Dietary Fibre | 4.183 | | |
| Ash | 4.950 | | |
| Total | 100.000 | 351.044 | 100.000 |

## Claims

1. A composition suitable for the relief of joint pain in athletes comprising a food grade anti-inflammatory substance and chondroitin sulphate or glucosamine.

2. A composition according to Claim 1, wherein the food grade anti-inflammatory substance is selected from the group comprising omega-3 fatty acids, ginger extract, green tea polyphenols and anti-inflammatory milk fractions.

3. A composition according to Claim 2 in which the anti-inflammatory milk fraction is a whey fraction.

4. A composition according to Claim 3 which comprises from 1 to 6 grams of the whey fraction.

5. A composition according to any preceding claim which comprises from 1 to 5 grams of glucosamine.

6. A composition according to any preceding claim which comprises from 1 to 8 grams of chondroitin sulphate.

7. A composition according to any preceding claim which is a nutritional composition.

8. A composition according to Claim 7 which is a cereal bar.

9. Use of a composition according to any one of Claims 1 to 6 in the manufacture of a medicament or therapeutic nutritional composition for the prevention or treatment of joint pain in athletes.
